## Europäisches Patentamt
## European Patent Office
## Office européen des brevets

(19)

(11) Numéro de publication: **0193420 B1**

(12)

# FASCICULE DE BREVET EUROPEEN

(45) Date de publication du fascicule du brevet:
28.12.88

(51) Int. Cl.⁴: **C 12 Q 1/00, C 12 M 1/40**

(21) Numéro de dépôt: 86400096.3

(22) Date de dépôt: 17.01.86

(54) **Dispositif pour le dosage des effecteurs de la chaîne d'oxydation cellulaire et procédés de dosage de ces effecteurs.**

(30) Priorité: 18.01.85 FR 8500728

(43) Date de publication de la demande:
03.09.86 Bulletin 86/36

(45) Mention de la délivrance du brevet:
28.12.88 Bulletin 88/52

(84) Etats contractants désignés:
AT BE CH DE FR GB IT LI LU NL SE

(56) Documents cités:
BIOTECHNOLOGY AND BIOENGINEERING, vol. 25, no. 11, novembre 1983, pages 2557-2566, John Wiley & Sons Inc., New York, US; J.L. ROMETTE et al.: "Enzyme electrode for specific determination of L-lysine"
TRAC TRENDS IN ANALYTICAL CHEMISTRY, vol. 2, no. 7, juillet 1983, pages 154-157, Elsevier Science Publishers B.V., Cambridge, GB; R.K. KOBOS: "Microbe-based electrochemical sensing systems"
ANALYTICAL CHEMISTRY, vol. 52, no. 7, juin 1980, pages 1020-1024, American Chemical Society, New York, US; M. HIKUMA et al.: "Ammonia electrode with immobilized nitrifying bacteria"

(73) Titulaire: **CENTRE NATIONAL DE LA RECHERCHE SCIENTIFIQUE (CNRS), 15, Quai Anatole France, F-75007 Paris (FR)**

(72) Inventeur: **Burstein, Claude, 21 Avenue du Bois Brandin, F-60580 Coye la Foret (FR)**
Inventeur: **Rabouille, Catherine, 61 Square du Nord, F-95500 Gonesse (FR)**
Inventeur: **Romette, Jean-Louis, Chemin de Cambronne, F-60129 Orrouy (FR)**
Inventeur: **Adamowicz, Elisabeth, 21 Avenue Claude Vellefaux, F-75010 Paris (FR)**
Inventeur: **Boucherit, Kébir, 55/57 Boulevard de Valmy, F-92700 Colombes (FR)**

(74) Mandataire: **Phélip, Bruno et al, c/o Cabinet Harlé & Phélip 21, rue de La Rochefoucauld, F-75009 Paris (FR)**

**Description**

La présente invention concerne un dispositif pour le dosage spécifique des substrats, inhibiteurs et activateurs de la chaîne d'oxydation cellulaire, mettant en jeu les réactions enzymatiques de la chaîne respiratoire et les procédés de dosage de ces composés.

On a déjà utilisé des capteurs électrochimiques associés à un film dans lequel était immobilisé une enzyme, pour doser des composés présents dans les milieux biologiques à des concentrations comprises entre 0,01 M à $10^{-5}$ M. George G. GUILBAULT a fait une revue des utilisations des enzymes immobilisées à des fins analytiques, qui a été publiée dans Applied Biochem. Biotechn. 7, 85—98 (1982). La plupart des électrodes à enzyme décrites jusqu'ici mettent en jeu une seule enzyme isolée de son milieu naturel et ayant subi différentes purifications. D'autres électrodes mettent en jeu des systèmes enzymatiques plus complexes, comme la L-lactate oxydase de Segmatis perfringens, enzyme nécessitant un coenzyme flavinique, ou la L-lactate deshydrogénase, avec son cofacteur $NAD^+$, ou encore les systèmes présents dans des bactéries entières.

On a maintenant trouvé que l'on pouvait utiliser pour le dosage sélectif de certains composés, un dispositif dans lequel est associé à un capteur d'oxygène non plus une enzyme préalablement purifiée, mais un système multi-enzymatique, à savoir celui intervenant dans les processus d'oxydation cellulaire formant la chaîne respiratoire, sans isoler préalablement ledit système de la cellule où il fonctionne et sans addition de coenzymes ou cofacteurs. Toutefois, dans ces conditions tous les effecteurs de la chaîne respiratoire, c'est-à-dire ses substrats, inhibiteurs, activateurs et produits sont dosés simultanément et ceci serait particulièrement gênant dans les cas où les milieux à étudier comprennent des mélanges de plusieurs de ces effecteurs, si une autre caractéristique de l'invention ne permettait d'éliminer cette difficulté et d'effectuer des dosages sélectifs. Elle consiste, pour éliminer les flavoprotéines parasites à utiliser les chaînes respiratoires de cellules qui ont été préalablement cultivées dans des milieux dont la composition a entraîné une modification au niveau de la flavoprotéine correspondant au composé à doser.

Certains milieux induisent la synthèse d'une flavoprotéine de la chaîne respiratoire; d'autres milieux comportant du glucose réprimeront la synthèse de certaines flavoprotéines.

On a constaté que les flavoprotéines correspondant au L-lactate, succinate, glycérol, fumarate ou L-malate sont inductibles respectivement en présence de lactate, succinate, glycérol ou malate pour les deux derniers. Ces mêmes flavoprotéines sont réprimées cataboliquement par le glucose, alors que les flavoprotéines correspondant au D-lactate, pyruvate, NADH ou NADPH ne sont pas touchées.

Parmi les effecteurs pouvant être dosés par le dispositif de la présente invention, on citera les acides D-lactique et L-lactique, pyruvique, succinique, fumarique, oxaloacétique, L-malique et leurs sels, le NADH et le NADPH ou encore les acides glutamique, aspartique, citrique, isocitrique, ascorbique, 3-glycéro-phosphorique, oxalique et leurs sels, acides dont le métabolisme conduit à des substrats de la chaîne respiratoire, ou encore les oxydoréductases dépendantes des cofacteurs à $NAD^+$ ou $NADP^+$.

Le présent dispositif comporte un capteur d'oxygène, associé à des moyens de mesure de l'oxygène capté, et l'ensemble des transporteurs de la chaîne respiratoire cellulaire qui se présente sous forme de bactéries immobilisées, microorganismes immobilisés ou d'une de leurs préparations membranaires immobilisée, ces cellules ayant été préalablement cultivées sur un milieu dont la composition a été choisie de telle sorte qu'il y ait une modification de la synthèse cellulaire de l'enzyme correspondant au composé à doser, c'est-à-dire de la flavoprotéine intervenant dans une réaction dudit composé.

Comme bactérie, on peut utiliser toutes les bactéries aérobies, comme Entérobactériaceae: Micrococcus et Paracoccus denitrificans, Azotobacter venelandi Bacillus ou des bactéries thermophiles comme $PS_3$; on préfère les entériobactériacées et particulièrement Escherichia coli; comme microorganismes, on peut citer les levures.

Le milieu de culture des bactéries pourra être quelconque dans le cas de la répression par le glucose du moment qu'au moins 1 g/l de glucose sont présents. Par contre, pour assurer l'induction de la synthèse d'une flavoprotéine, on utilisera un milieu de culture dit «minimum» complémenté par l'agent modificateur convenable.

Par milieu minimum, on entend un milieu aéré liquide comportant les minéraux nécessaires au développement cellulaire. Dans le cas d'Escherichia coli, on utilise plus particulièrement le milieu contenant 10,6 g/l de $KH_2PO_4$, 174 g/l de $K_2HPO_4$, 4 g/l de $(NH_4)_2SO_4$, 0,4 g/l de $MgSO_4 \cdot 7H_2O$, 0,001 g/l de $FeSO_4 \cdot 7H_2O$, auquel on ajoute 1 mg/l de thiamine. La concentration de l'agent modificateur dans ce milieu n'est pas critique; toutefois, comme le rendement de la culture en dépend, on préfère en introduire de 4 g/l à 10 g/l.

A titre de modificateur, pour le dosage des acides L-lactique et pyruvique ou de leurs sels, on utilisera un lactate et notamment un racémique; pour le dosage de l'acide 3-glycérophosphorique ou pyruvique, on utilisera le glycérol; pour le dosage des acides succinique et fumarique ou de leurs sels, on utilisera un succinate; pour le dosage des acides L-malique et oxaloacétique ou de leurs seuls, un malate et notamment un racémique.

Pour le dosage de l'acide pyruvique, de l'acide D-lactique ou de leurs sels ou du NADH ou du NADPH, on utilisera, comme modificateur dans un milieu de culture quelconque le glucose qui réprime la synthèse des flavoprotéines inductibles citées ci-dessus.

Pour doser les composés qui n'interviennent pas directement au niveau de la chaîne respiratoire, mais y aboutissent par voie métabolique, les cultures bactériennes se feront en présence de l'agent modificateur correspondant au composé par lequel ils entrent dans la chaîne respiratoire. Par exemple, pour le dosage de l'acide oxalique, les bactéries auront été cultivées sur milieu minimum en présence de succinate.

Les cellules ou les fragments cellulaires sont immobilisés par des moyens classiques pour former un ensemble insoluble en milieu aqueux. On utilise de préférence la gélatine; les cellules et fragments cellulaires sont incorporés dans la gélatine et le film obtenu est tanné par action d'un agent bifonctionnel, tel que le glutaraldéhyde.

Dans le cas des bactéries entières ou non, une préparation de cellules immobilisées contiendra de 10 à 40 mg de protéines bactériennes, sous la forme de bactéries, isolées du milieu de culture convenable pour 30 à 70 mg de gélatine. Un film utilisable dans un dispositif selon l'invention, sera préparé par étalement de 1 ml d'une suspension dans l'eau de bactéries, correspondant à 10 à 40 mg de protéines, contenant 30 à 70 mg de gélatine, vers 40°C, sur une surface de 40 cm² environ, puis par séchage dans l'air, suivi de l'introduction du film ainsi obtenu pour quelques minutes, dans une solution aqueuse de glutaraldéhyde de concentration comprise entre 1 et 4% (p/v) maintenue vers 0°C, suivie par un lavage abondant avec une solution tamponée. Pour notamment augmenter la tenue du film de bactéries immobilisées, on préfère étaler la suspension aqueuse de bactéries et de gélatine sur un film souple de polymère hydrophobe, mais perméable aux gaz, tel que le polypropylène et les deux films solidaires seront utilisés dans le dispositif de l'invention.

Pour le dosage sélectif de l'acide pyruvique ou de ses sels, du NADH et du NADPH, on perméabilise les membranes des bactéries avant résulter d'un traitement chimique, par le toluène, d'un traitement par les ultra-sons ou consistant en congélations et décongélations successives ou en une compression obtenue au moyen de la presse de French, suivie d'une décompression brutale.

Par ailleurs, pour permettre un dosage sélectif de l'acide succinique ou de ses sels en présence des autres substrats de la chaîne respiratoire et particulièrement en présence de quantités de lactate qui interfèrent, le dispositif selon l'invention peut être préparé avec des bactéries qui ont été soumises à une élévation de température avant leur immobilisation. Dans le cas d'Escherichia coli, ce traitement résulte d'une exposition à une température comprise entre 53°C et 57°C environ pendant 1 à 2 heures. On applique le même traitement pour préparer un dispositif utile au dosage sélectif de l'acide pyruvique ou de ses sels, en présence de NADH ou NADPH.

A la lecture de ce qui précède, on comprend que le choix d'une certaine combinaison des différents moyens cités de réalisation des dispositifs selon l'invention permet de préparer des capteurs convenant à des dosages particulièrement sélectifs, dans des milieux contenant des mélanges d'effecteurs.

Comme capteur à oxygène, on peut utiliser notamment une électrode à oxygène, du type dit «de Clark», une sonde céramique en zircone ou des transistors à effet de champ (FET).

On préfère comme dispositif selon l'invention une électrode à oxygène, sur laquelle a été déposé un film de bactéries immobilisées dans la gélatine, de 0,05 mm à 0,5 mm d'épaisseur. On a montré que dans ces conditions l'oxygène mesuré n'est pas celui consommé dans l'ensemble de l'échantillon mais celui qui a été préalablement stocké dans le support; ainsi le résultat de la mesure est indépendant du degré d'oxygénation du milieu à étudier. Dans une forme de réalisation préférée, on dispose entre l'électrode à oxygène et le film enzymatique, un film hydrophobe qui ne laissera passer que l'oxygène et par exemple un film de polypropylène.

Le dispositif préféré de l'invention, constitué d'une électrode à oxygène et d'un film enzymatique, y adhérant éventuellement par l'intermédiaire d'un autre film, est très sensible et convient particulièrement bien aux dosages directs dans des milieux biologiques, sans traitement préalable. On a constaté que lorsque le film au lieu d'être appliqué sur l'électrode est simplement mis en suspension dans le compartiment électrode contenant l'échantillon à doser, le signal mesurable, pour une même concentration de produit à doser et de matériel biologique immobilisé, est de 100 à 600 fois plus faible. Un tel dispositif est en outre fidèle, puisque les résultats des mesures sont identiques si l'on effectue plusieurs centaines de dosages même à des temps rapprochés puisqu'il peut être conservé vers 4°C en milieu aqueux tamponné en présence d'un antibactérien pendant plus d'un an, sans altération de ses propriétés.

Dans Biotechnol. Bioeng. XXV p. 2557–2566 (1983), on décrit un dispositif pour le dosage de la L-lysine comportant une électrode à enzyme et des moyens de mesure de la quantité d'oxygène consommée; même si, dans ce cas, l'électrode comporte une seule enzyme la lysineoxydase qui e été isolée puis immobilisée, l'électrode à oxygène et les moyens de mesure qui y sont associés conviennent à la préparation d'un dispositif selon l'invention et on pourra se référer à ce document, cité dans l'invention à titre de référence, pour la description d'une forme de réalisation d'un dispositif selon l'invention, sachant qu'en substituant au film monoenzymatique de l'art antérieur un film contenant le système des transporteurs de la chaîne respiratoire cellulaire, tel que décrit ici, on obtiendra un exemple d'électrode à enzyme de l'invention.

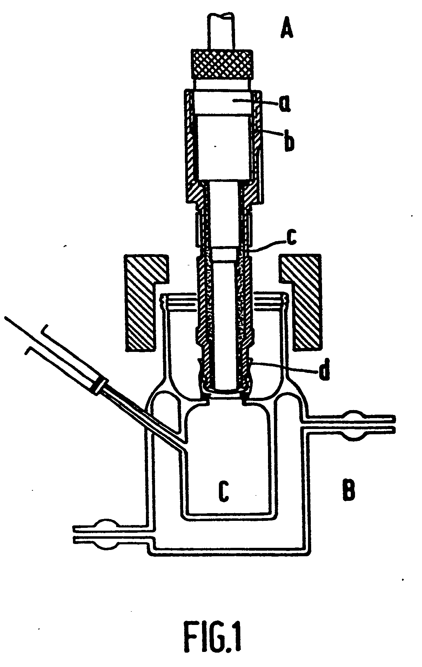

La Fig. 1 représente une électrode A et sa cellule de mesure B dans laquelle l'échantillon est introduit en C; a désigne le corps de l'électrode et b son enveloppe, c l'électrolyte et d le film à enzyme qui est appliqué sous le bout de l'électrode. L'électrode a environ 1 cm de diamètre tandis que la partie terminale sensible a 7 mm de diamètre. La cellule de mesure C contient 1 ml d'échantillon.

D'autres moyens de mesure de la quantité d'oxygène peuvent être les moyens classiques, habituellement utilisés avec les capteurs compris dans le dispositif.

Un autre objet de l'invention est constitué des procédés de dosage des effecteurs de la chaîne respiratoire dans des milieux divers, qui mettent en œuvre les dispositifs précédement décrits. L'application de ces precédés de dosage ne nécessite pas de traitement préalable de ces milieux, tels que dialyse ou filtration, pour éliminer d'éventuels produits gênants, ce qui est un autre avantage de l'invention. Parmi les milieux dans lesquels ces dosages peuvent être effectués, on peut citer les liquides biologiques, tels que urine, sang ou plasma, liquide céphalo-rachidien, mais aussi des produits de l'industrie agro-alimentaire, comme les yaourts, les desserts lactés, le vin, les jus de fruits et la bière.

Les dispositifs seront en général réalisés de telle sorte qu'ils permettent le dosage des composés lorsqu'ils sont présents dans le milieu à étudier à une concentration comprise entre 0,1 mM et 100 mM.

Les procédés de dosage selon l'invention reposent sur la détermination de la quantité d'oxygène consommée par le système de transporteurs à oxydo-réduction de la chaîne respiratoire, lors de la transformation de l'effecteur à doser. La mesure de l'oxygène consommé est effectuée à l'aide d'un dispositif selon l'invention, couplé aux moyens électroniques habituels de quantification.

Pour le dosage sélectif des acides, D-ou L-lactique ou de ses sels en présence des autres substrats de la chaîne respiratoire, et tout particulièrement en présence de succinate qui pourrait interférer, selon l'invention on n'effectue la mesure qu'après avoir ajouté du fumarate ou du malonate dans le milieu de dosage, ce qui inhibe la respiration du succinate; pour une concentration d'acide lactique dans le milieu à doser comprise entre 1 mM et 10 mM, on introduit de 100 mM à 300 mM de fumarate ou de malonate dans le milieu.

Pour le dosage sélectif de l'acide succinique ou de ses sels en présence des autres substrats de la chaîne respiratoire et tout particulièrement en présence de lactate qui interfère, selon l'invention on ajoute du pyruvate dans le milieu de dosage, avant la mesure; pour une concentration d'acide succinique dans le milieu à doser comprise entre 2 mM et 10 mM, on introduit de 100 mM à 300 mM de pyruvate.

Dans ce qui suit, on décrit la préparation de certains dispositifs conformes à l'invention ainsi que des procédés de dosage les mettant en œuvre.

Exemple 1: Préparation d'un dispositif
a) Culture des bactéries:
1. Entraînant l'induction de la synthèse d'une flavoprotéine: Escherichia coli K12, de la souche 3300, a été cultivé à 37°C sur un milieu minimum minéral aéré (référence n° 63, concentré 2 fois)

contenant 10,6 g/l de $KH_2PO_4$, 174 g/l de $K_2HPO_4$, 4 g/l de $(NH_4)_2SO_4$, 0,4 g/l de $MgSO_4 \cdot 7H_2O$, 0,001 g/l de $FeSO_4 \cdot 7H_2O$ et 1 mg/l de thiamine. On a introduit dans le milieu à chaque fois 10 g/l de modificateur. Quatre cultures différentes ont été effectuées, comportant chacune un modificateur différent: lactate (racémique), succinate, malate (racémique), glycérol.

Les bactéries ont été récoltées en phase exponentielle par centrifugation et remises en suspension dans le même milieu minimum, sans modificateur ni autre source de carbone, à raison de 1 à 10 mg de protéines bactériennes par ml de suspension. Après agitation violente, à 37°C, pendant 1 heure pour éliminer la plupart des substrats endogènes respiratioires, on a lavé les bactéries trois fois avec un tampon phosphate de potassium (0,2 M, pH = 7,8), et on les a remis en suspension, à raison de 10 à 40 mg de protéines bactériennes par ml dans un tampon phosphate de K (0,05 M, pH = 7,8) avant de les congeler à −80°C pour les conserver. Avant utilisation, on effectue une décongélation rapide à 37°C. L'activité respiratoire n'est pas modifiée après une conservation à −80°C pendant plus d'un an.

2. Entraînant une répression catabolique: Escherichia coli est cultivé sur un des milieux de culture habituels, minimum ou riche, auquel on a ajouté 10 g/l de glucose.

On peu aussi cultiver à 65°C la souche thermophile PS3 dans un milieu richte contenant 10 g/l de glucose.

La récolte, l'isolement et la conservation des bactéries ainsi cultivées se font comme précédement.

b) Procédé de perméabilisation des membranes bactériennes: Cette étape n'est nécessaire que lors de la préparation d'un dispositif convenant au dosage de NADH, NADPH, acide pyruvique et ses sels.

La suspension bactérienne préalablement obtenue est traitée par l'une des trois méthodes qui suivent pour perméabiliser les membranes bactériennes.

– Action des ultrasons: de 2 à 5 ml de suspension bactérienne sont soumis 4 fois durant 10 secondes, à 0°C, à l'action d'un appareil à ultrasons MSE® comportant une sonde de 0,6 cm de diamètre.

– Méthode de la presse de French: la suspension bactérienne est passée dans un fractionateur de cellules à 115°C et sous une pression de 1600 bars. Les bactéries entières et les fragments de membrane externe sont éliminés par une centrifugation à 60 000 g, pendant 20 minutes.

– Choc thermique: on effectue 6 congélations/décongélations successives entre −80°C et +37°C.

c) Immobilisation: Les bactéries, perméabilisées ou non, sont mélangées à 40°C avec une solution aqueuse de gélatine à raison de 20 mg de protéines bactériennes et de 50 mg de gélatine pour 1 ml de suspension finale. La gélatine, de 250 blooms, est de la gélatine d'os ou de peau de porc commercialisée par la société Rousselot (France). Le mélange est homogénéisé puis étalé rapidement sur un film de polypropylène, d'épaisseur 6 μm, commercialisé par la société Bollore (France). On prépare ainsi 40 cm² de film par ml de mélange. Après 5 minutes à 4°C ou une nuit à 4°C, le film est tanné par trempage de l'ensemble à 0°C, dans une solution aqueuse à 2% (p/V) de glutaraldéhyde pendant 3 minutes.

Le film est alors immédiatement lavé 3 fois avec une solution aqueuse de lysine (0,1 M, pH $\psi$ 7,6), puis 3 fois avec un tampon phosphate (50 mM, pH $\psi$ 7,6) et lysine (50 mM, pH $\psi$ 7,6).

Le film peut être conservé à 4°C, en l'absence d'oxygène, dans ce dernier tampon contenant en outre 0,5 mM d'azoture de sodium.

d) Préparation d'une électrode à enzyme: On recouvre la zone sensible à l'oxygène d'une électrode de Clark qui fait environ 10 mm², avec 2 cm² environ du film enzymatique à support de polypropylène précédemment obtenu, le polypropylène étant au contact de l'électrode. Pour la mesure, l'électrode est reliée à un oxygraphe automatique, du type de celui décrit dans Biotechnol. Bioeng. précédemment cité.

Exemple 2: Procédé de dosage

a) Méthode: On effectue successivement:
— le rinçage de l'électrode avec un tampon phosphate de K (0,1 M − pH 5,5) ou un tampon acétate de K (0,1 M−pH 5,5) dans le cas du dosage de NADPH;
— la mise de l'électrode à l'air, quelques secondes pour la saturer en oxygène;
— l'introduction de l'échantillon à doser dans le compartiment de l'électrode, en maintenant la température à 30°C;
— la mesure de la pente au point d'inflexion du signal électrique, ce qui donne la vitesse initiale de la consommation d'oxygène;
— le retrait de l'échantillon du compartiment de l'électrode et son rinçage avec le même tampon que précédemment pour saturer de nouveau en oxygène le film de gélatine.

b) Résultats

Dans ces conditions, on peut déterminer la concentration d'un substrat dans un milieu en mesurant la «vitesse initiale» et en la comparant à celle figurant sur la courbe d'étalonnage, tracée préalablement. Cette comparaison peut être faite automatiquement par un appareillage convenable connu de l'homme de métier.

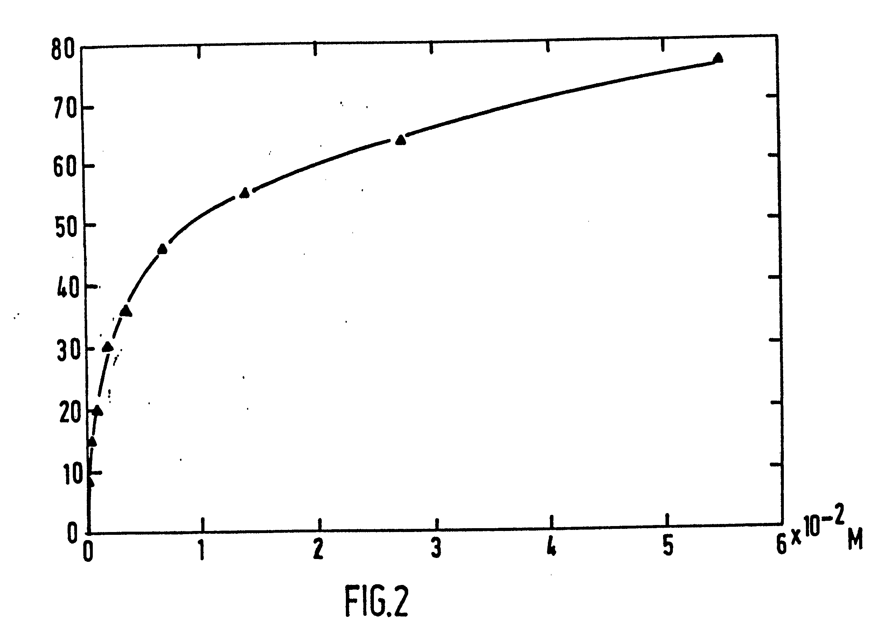

On a représenté Fig. 2 la courbe d'étalonnage obtenue avec une électrode à enzyme, préparée avec Escherichia coli cultivé en présence de lactate, obtenue pour du L-lactate de Na en solution aqueuse, à une concentration inférieure à 0,06 M. En abscisse, sont portées les concentrations et en ordonnées la quantité d'oxygène consommée en μ-moles. Dans le cas où du succinate est présent dans l'échantillon de L-lactate à doser, ou risque d'être présent, on introduit préalablement à la mesure, un volume connu d'une solution aqueuse de fumarate de sodium ou malonate de sodium, de telle sorte que la concentration finale soit de 100 mM à 300 mM; la courbe d'étalonnage n'est pas modifiée. On dose ainsi l'acide L-lactique ou ses sels, à des concentrations comprises entre 1 et 40 mM.

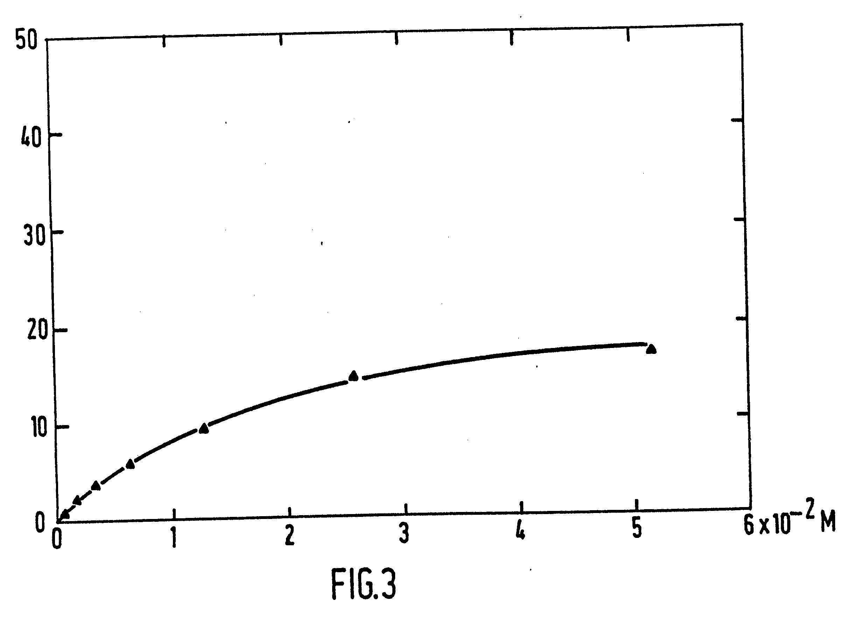

— On dose ainsi l'acide succinique, ou ses sels, à des concentrations comprises entre 1 mM et 40 mM avec une électrode préparée avec Escherichia coli cultivé en présence de succinate. La courbe d'étalonnage obtenue est représentée Fig. 3. Dans les cas où du lactate est présent dans l'échantillon à doser, ou risque de l'être, on y introduit préalablement à la mesure du pyruvate, à raison de 100 mM à 300 mM. On peut aussi utiliser des bactéries chauffées à 57°C pendant 2 heures, avant leur immobilisation, ce qui n'a aucune influence sur la «respiration» du succinate mais diminue d'un facteur supérieur à 10 celle du lactate.

— On dose ainsi l'acide L-malique, ou ses sels, à des concentrations comprises entre 1 mM et 40 mM, avec une électrode préparée à partir d'Escherichia coli cultivées sur un milieu minimum en présence de malate. L'addition de malonate et de pyruvate, à une concentration de 100 à 300 mM dans l'échantillon à doser, permet d'éviter que les résultats ne soient faussés par la présence simultanée dans l'échantillon respectivement d'acide succinique ou lactique, ou de leurs sels.

— On dose ainsi l'acide D-lactique ou ses sels, à des concentrations comprises entre 1 mM et 40 mM, avec une électrode préparée avec Escherichia coli cultivé en présence de glucose. Dans ce cas encore, l'addition de fumarate et/ou de malonate dans l'échantillon, élimine les conséquences sur le dosage de la présence d'acide succinique ou de ses sels.

— On dose ainsi le NADH ou le NADPH à des concentrations comprises entre 0,1 mM et 10 mM en préparant l'électrode avec Escherichia coli cultivé à 37°C sur un milieu riche en présence de glucose ou avec des $PS_3$ cultivées à 65°C, et perméabilisées avant immobilisation. Pour le NADH, il est préférable d'ajuster le pH de l'échantillon vers 7,6, et pour le NADPH vers 5,8. Le dosage des déshydrogénases à cofacteurs $NAD^+$ et $NADP^+$ s'effectue de la même façon.

— On dose ainsi l'acide fumarique ou ses sels à des concentrations comprises entre 1 mM et 40 mM avec une électrode préparée avec des Escherichia coli cultivé sur le milieu minimum en présence de malate, ou succinate. Dans le cas du succinate, on ajoutera du succinate dans le milieu à doser. L'introduction de pyruvate dans l'échantillon à doser, à une concentration comprise entre 100 et 300 mM élimine le risque d'interférence avec les dérivés lactiques pouvant être présents dans l'échantillon.

— On dose ainsi l'acide oxalique ou ses sels avec une électrode préparée avec Escherichia coli cultivé sur un milieu minimum contenant 10 g/l de succinate, ou encore l'acide 3-glycérophosphorique avec une électrode préparée avec Escherichia coli cultivé sur un milieu minimum contenant 10 g/l de glycérol.

— On dose ainsi l'acide pyruvique, ou ses sels, à des concentrations comprises entre 2 mM et

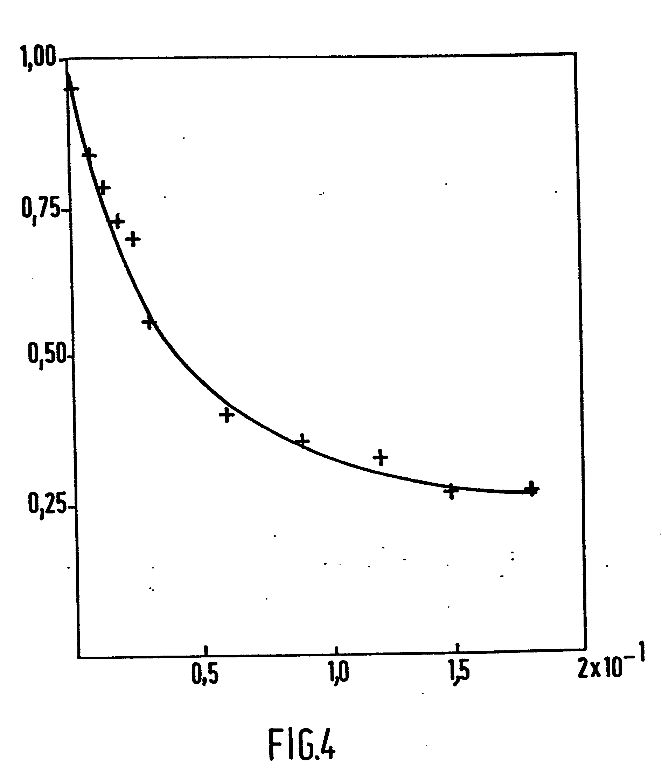

100 mM avec une électrode préparée avec Escherichia coli cultivé en présence de lactate. Le dosage doit alors être effectué en présence de L-lactate; pour une concentration en pyruvate à doser inférieure à 0,2 mM, on ajoute dans l'échantillon avant la mesure une quantité de L-lactate suffisante pour obtenir une concentration 5 mM. La Fig. 4 représente la courbe d'étalonnage obtenue pour le pyruvate de sodium en solution aqueuse.

On peut aussi doser l'acide pyruvique, ou ses sels avec une électrode préparée avec Escherichia coli perméabilisé, cultivé sur un milieu minimum contenant 10 g/l glycérol, ou sur un milieu quelconque contenant du glucose.

Dans le cas où l'échantillon peut contenir du NADH, NADPH ou du D-lactate, le maintien des bactéries à 57°C pendant 1 heure; avant leur immobilisation, permet de diminuer les risques de perturbation des résultats du dosage, puisque l'activité «respiratoire» de ces 2 composés décroît alors d'un facteur supérieur à 10, sans que la «respiration» du pyruvate soit modifée.

**Revendications**

1. Dispositif pour le dosage spécifique de substrats, inhibiteurs ou activateurs de la chaîne d'oxydation cellulaire, caractérisé en ce qu'il comporte un capteur d'oxygène, associé à des moyens de mesure de l'oxygène capté, et les transporteurs de la chaîne respiratoire cellulaire sous forme de bactéries immobilisées, de microorganismes immobilisés ou de leurs préparations membranaires immobilisées, ces cellules ayant été préalablement cultivées sur un milieu dont la composition a entraîne une modification de la synthèse cellulaire de la flavoprotéine correspondant au composé à doser.

2. Dispositif selon la revendication 1, caractérisé par le fait que les effecteurs à doser sont choisis parmi les acides D-lactique et L-lactique, pyruvique, succinique, fumarique, oxaloacétique, L-malique, et leurs sels, le NADH et NADPH, ou encore les acides glutamique, aspartique, citrique, isocitrique, ascorbique, 3-glycero-phosphorique, oxalique et leurs sels, ou encore les oxydo-réductases dispentantes des cofacteurs à $NAD^+$ on $NADP^+$.

3. Dispositif selon l'une des revendications 1 et 2 caractérisé en ce que le milieu de culture des cellules a induit la synthèse de la flavoprotéine.

4. Dispositif selon la revendication 3, caractérisé en ce que l'induction résulte de la présence dans le milieu de culture minimum d'une seule source de carbone, qui est le substrat de la chaîne respiratoire correspondant à la flavoprotéine.

5. Dispositif selon la revendication 1, caractérisé en ce que le milieu de culture contient du glucose pour réprimer certaines flavoprotéines.

6. Dispositif selon l'une quelconque des revendications précédentes, caractérisé en ce que l'immobilisation a lieu dans de la gélatine avec un tannage par action du glutaraldéhyde.

7. Dispositif selon la revendication 6, caractérisé en ce que les cellules sont immobilisées dans un film préparé à partir d'une suspension de cellules comportant 10 à 40 mg de protéines bactériennes et 50 mg de gélatine par ml. étalé sur 40 cm², séché puis tanné par action d'une solution aqueuse de glutaraldéhyde de concentration comprise entre 1 à 4% (p/v) pendant quelques minutes.

8. Dispositif selon l'une des revendications précédentes, caractérisé en ce qu'il est constitué d'une électrode à oxygène sur laquelle a été déposé le film de cellules ou préparations cellulaires immobilisées.

9. Dispositif selon l'une des revendications 1 à 4 ou 6 à 8 pour le dosage de l'acide L-lactique, de l'acide pyruvique ou de leurs sels, caractérisé en ce qu'il comporte des bactéries immobilisées issues d'un milieu de culture minimum comportant un lactate comme seule source de carbone.

10. Dispositif selon l'une des revendications 1 à 4 ou 6 à 8 pour le dosage de l'acide succinique, de l'acide fumarique ou de leurs sels, caractérisé en ce que les bactéries ont été cultivées sur un milieu minimum comportant un succinate comme seule source de carbone.

11. Dispositif selon la revendication 10 pour le dosage de l'acide succinique ou de ses sels en présence des autres substrats de la chaîne respiratoire caractérisé en ce que Escherichia coli est soumis à une température de 53°C à 57°C, pendant 1 à 2 heures, avant immobilisation.

12. Dispositif selon l'une des revendications 1 à 4 ou 6 à 8 pour le dosage de l'acide L-malique, de l'acide oxaloacétique ou fumarique ou de leurs sels, caractérisé en ce que les bactéries ont été cultivées sur un milieu minimum comportant un malate comme seule source de carbone.

13. Dispositif selon l'une des revendications 5 à 8 pour le dosage de l'acide D-lactique ou de ses sels, caractérisé en ce que les bactéries ont été cultivées sur un milieu comportant du glucose.

14. Dispositif selon l'une des revendications 5 à 8 pour le dosage de l'acide pyruvique ou de ses sels, du NADH ou du NADPH, caractérisé en ce que les bactéries proviennent d'un milieu de culture comportant du glucose et en ce que les membranes des bactéries ont été perméabilisées avant leur immobilisation.

15. Dispositif selon la revendication 14 pour la dosage de l'acide pyruvique ou de ses sels en présence des autres substrats de la chaîne respiratoirs caractérisé en ce que Escherichia coli sont soumises à une température de 53°C à 57°C, pendant 1 à 2 heures, avant la perméabilisation de leurs membranes.

16. Dispositif selon l'une des revenciations 1 à 4 ou 6 à 8 pour le dosage de l'acide 3-glycérophosphorique, ou pyruvique caractérisé en ce que les bactéries ont été cultivées sur un milieu minimum comportant du glycérol comme seule source de carbone.

17. Procédé de dosage spécifique des substrats, inhibiteurs et activateurs de la chaîne d'oxydation cellulaire, caractérisé en ce que l'on détermine la quantité d'oxygène consommée par le système de transporteurs constituant la chaîne respiratoire pour transformer le produit à doser, le système étant introduit dans le milieu à étudier sous forme de bactéries immobilisées, de micro-organismes immobilisés ou d'une de leurs préparations membranaires immobilisées, ces cellules ayant été préalablement cultivées dans des milieux dont la composition a entraîné une modification de la synthèse cellulaire de la flavoprotéine correspondant au composé à doser.

18. Procédé selon la revendication 17, caractérisé par le fait que les effecteurs à doser sont choisis parmi les acides D-lactique et L-lactique, pyruvique, sucinique, fumarique, oxaloacétique, L-malique et leurs sels, le NADH et le NADPH, ou encore les acides glutamique, aspartique, citrique, isocitrique, ascorbique, 3-glycero-phosphorique, oxalique et leurs sels, ou encore des oxydo-réductases dépendantes des cofacteurs à NAD$^+$ ou NADP$^+$.

19. Procédé de dosage selon la revendication 16, mettant en œuvre un dispositif selon l'une quelconque des revendications 1 à 16.

20. Procédé selon la revendication 17 pour le dosage de l'acide L-lactique ou de ses sels en présence d'autres substrats pouvant interférer de la chaîne respiratoire avec un dispositif selon la revendication 9, caractérisé en ce qu'on introduit dans le milieu de dosage du fumarate ou du malonate.

21. Procédé selon la revendication 17 pour le dosage de l'acide D-lactique ou de ses sels en présence d'autres substrats pouvant interférer de la chaîne respiratoire, avec un dispositif selon la revendication 13, caractérisé en ce qu'on introduit dans le milieu de dosage du fumarate ou du malonate.

22. Procédé selon la revendication 17 pour le dosage de l'acide succinique ou de ses sels en présence d'autres substrats pouvant interférer de la chaîne respiratoire avec un dispositif selon la revendication 11, caractérisé en ce qu'on introduit dans le milieu de dosage du pyruvate.

23. Procédé selon la revendication 17 pour le dosage de l'acide L-malique ou de ses sels en présence d'autres substrats pouvant interférer de la chaîne respiratoire avec un dispositif selon la revendication 12, caractérisé en ce qu'on introduit dans le milieu de dosage du malonate et/ou du pyruvate.

24. Procédé selon la revendication 17 pour le dosage de l'acide pyruvique ou de ses sels avec un dispositif selon la revendication 9, caractérisé en ce qu'on introduit dans le milieu de dosage du L-lactate.

25. Procédé selon la revendication 17 pour le dosage de l'acide fumarique ou de ses sels en présence d'autres substrats pouvant interférer de la chaîne respiratoire avec un dispositif selon la revendication ou 12, caractérisé en ce qu'on introduit dans le milieu de dosage du pyruvate.

**Patentansprüche**

1. Vorrichtung zur spezifischen Bestimmung von Substraten, Inhibitoren oder Aktivatoren der zellularen Oxidationskette, dadurch gekennzeichnet, dass sie einen Sauerstoffeinfänger in Verbindung mit Mitteln zur Messung des eingefangenen Sauerstoffs und die Transporteure der Zellatmungskette in Form von immobiliserten Bakterien, immobilisierten Mikroorganismen oder ihren immobilisierten Membranpräparaten aufweist, wobei diese Zellen vorher auf einem Medium, dessen Zusammensetzung eine Modifikation der Zellsynthese des der zu bestimmenden Verbindung entsprechenden Flavoproteins im Gefolge hatte, kultiviert worden sind.

2. Vorrichtung nach Anspruch 1, dadurch gekennzeichnet, dass die zu bestimmenden Effektoren ausgewählt sind unter den D-Milch- und L-Milch-, Brenztrauben-, Bernstein-, Fumar-, Oxaloessig-, L-Äpfelsäuren und ihren Salzen, NADH und NADPH oder auch den Glutamin-, Asparagin-, Citronen-, Isocitronen-, Ascorbin-, 3-Glycero-phosphor-, Oxalsäuren und ihren Salzen oder auch den von den NAD$^+$- oder NADP$^+$-Cofaktoren abhängigen Oxidoreduktasen.

3. Vorrichtung nach einem der Ansprüche 1 und 2, dadurch gekennzeichnet, dass das Kulturmedium der Zellen die Synthese des Flavoproteins ausgelöst hat.

4. Vorrichtung nach Anspruch 3, dadurch gekennzeichnet, dass die Auslösung die Folge der Anwesenheit wenigstens einer einzigen Kohlenstoffquelle im Kulturmedium ist, die das dem Flavoprotein entsprechende Substrat der Atmungskette ist.

5. Vorrichtung nach Anspruch 1, dadurch gekennzeichnet, dass das Kulturmedium Glucose zur Unterdrückung gewisser Flavoproteine enthält.

6. Vorrichtung nach irgendeinem der vorstehenden Ansprüche, dadurch gekennzeichnet, dass die Immobilisierung in Gelatine mit einer Gerbung durch Einwirkung des Glutaraldehyds stattfindet.

7. Vorrichtung nach Anspruch 6, dadurch gekennzeichnet, dass die Zellen in einem Film immobilisert werden, der aus einer Suspension von Zellen, die 10 bis 40 mg bakterielle Proteine und 50 mg Gelatine pro ml enthält, hergestellt, auf 40 cm$^2$ ausgebreitet, getrocknet und dann durch Einwirkung einer wässrigen Lösung von Glutaraldehyd einer Konzentration zwischen 1 und 4% (Gew.-/Vol.) während einiger Minuten gegerbt worden ist.

8. Vorrichtung nach irgendeinem der vorstehenden Ansprüche, dadurch gekennzeichnet, dass sie aus einer Sauerstoffelektrode besteht, auf die der Film der immobiliserten Zellen oder Zellpräparate aufgetragen worden ist.

9. Vorrichtung nach irgendeinem der Ansprüche 1 bis 4 oder 6 bis 8 zur Bestimmung von L-Milchsäure, Brenztraubensäure oder ihrer Salze, dadurch gekennzeichnet, dass sie immobilisierte Bakterien umfasst, die aus einem Kulturmedium stammen, das minimalen ein Laktat als einzige Kohlenstoffquelle enthält.

10. Vorrichtung nach irgendeinem der Ansprüche 1 bis 4 oder 6 bis 8 zur Bestimmung von Bernsteinsäure, Fumarsäure oder ihren Salzen, dadurch gekennzeichnet, dass die Bakterien auf einem minimalen Medium, das ein Succinat als einzige Kohlenstoffquelle enthält, kultiviert worden sind.

11. Vorrichtung nach Anspruch 10 zur Bestimmung von Bernsteinsäure oder ihrer Salze in Gegenwart der anderen Substrate der Atmungskette, dadurch gekennzeichnet, dass Escherichia coli vor der Immobilisation während 1 bis 2 Stunden einer Temperatur von 53°C bis 57°C ausgesetzt wird.

12. Vorrichtung nach irgendeinem der Ansprüche 1 bis 4 oder 6 bis 8 zur Bestimmung von L-Äpfelsäure, Oxaloessigsäure oder Fumarsäure oder ihrer Salze, dadurch gekennzeichnet, dass die Bakterien auf einem minimalen Medium, das ein Malat als einzige Kohlenstoffquelle enthält, kultiviert worden sind.

13. Vorrichtung nach irgendeinem der Ansprüche 5 bis 8 zur Bestimmung von D-Milchsäure oder ihren Salzen, dadurch gekennzeichnet, dass die Bakterien auf einem Glucose enthaltenden Medium kultiviert worden sind.

14. Vorrichtung nach einem der Ansprüche 5 bis 8 zur Bestimmung von Brenztraubensäure oder ihrer Salze, von NADH oder NADPH, dadurch gekennzeichnet, dass die Bakterien von einem Glucose enthaltenden Kulturmedium stammen und dass die Bakterienmembranen vor ihrer Immobilisation durchlässig gemacht worden sind.

15. Vorrichtung nach Anspruch 14 zur Bestimmung von Brenztraubensäure oder ihrer Salze in Gegenwart der anderen Substrate der Atmungskette, dadurch gekennzeichnet, dass Escherichia coli vor der Permeabilisierung ihrer Membranen einer Temperatur von 53°C bis 57°C während 1 bis 2 Stunden ausgesetzt worden ist.

16. Vorrichtung nach einem der Ansprüche 1 bis 4 oder 6 bis 8 für die Bestimmung der 3-Glycerophosphorsäure oder Brenztraubensäure, dadurch gekennzeichnet, dass die Bakterien auf einem minimalen Medium, das Glycerin als einzige Kohlenstoffquelle enthält, kultiviert worden sind.

17. Verfahren zur spezifischen Bestimmung von Substraten, Inhibitoren und Aktivatoren der zellularen Oxidationskette, dadurch gekennzeichnet, dass man die Sauerstoffmenge bestimmt, die von dem die Atmungskette bildenden System von Transporteuren verbraucht worden ist, um das zu bestimmende Produkt umzuwandeln, wobei das System in das zu untersuchende Medium in Form von immobiliserten Bakterien, immobiliserten Mikroorganismen oder einem ihrer immobiliserten Membranpräparate eingeführt worden ist und diese Zellen vorher in einem Medium kultiviert worden sind, dessen Zusammensetzung eine Modifikation der Zellsynthese des der zu bestimmenden Verbindung entsprechenden Flavoproteins im Gefolge gehabt hat.

18. Verfahren nach Anspruch 17, dadurch gekennzeichnet, dass die zu bestimmenden Effektoren ausgewählt werden unter den D-Milch- und L-Milch-, Brenztrauben-, Bernstein-, Fumar-, Oxaloessig-, L-Äpfelsäuren und ihren Salzen, NADH und NADPH oder auch den Glutamin-, Asparagin-, Citronen-, Isocitronen-, Ascorbin-, 3-Glycero-phosphor-, Oxalsäuren und ihren Salzen oder auch den von den NAD⁺-, oder NADP⁺-Cofaktoren abhängigen Oxidoreduktasen.

19. Bestimmungsverfahren nach Anspruch 17 unter Verwendung einer Vorrichtung nach irgendeinem der Ansprüche 1 bis 16.

20. Verfahren nach Anspruch 17 zur Bestimmung der L-Milchsäure oder ihrer Salze in Gegenwart anderer Substrate der Atmungskette, die stören können, mit einer Vorrichtung nach Anspruch 9, dadurch gekennzeichnet, dass man Fumarat oder Malonat in das Bestimmungsmedium einführt.

21. Verfahren nach Anspruch 17 zur Bestimmung von D-Milchsäure oder ihrer Salze in Gegenwart anderer Substrate der Atmungskette, die stören können, mit einer Vorrichtung nach Anspruch 13, dadurch gekennzeichnet, dass man Fumarat oder Malonat in das Bestimmungsmedium einführt.

22. Verfahren nach Anspruch 17 zur Bestimmung von Bernsteinsäure oder ihrer Salze in Gegenwart anderer Substrate der Atmungskette, die stören können, mit einer Vorrichtung nach Anspruch 11, dadurch gekennzeichnet, dass man Pyruvat in das Bestimmungsmedium einführt.

23. Verfahren nach Anspruch 17 zur Bestimmung von L-Äpfelsäure oder ihrer Salze in Gegenwart anderer Substrate der Atmungskette, die stören können, mit einer Vorrichtung nach Anspruch 12, dadurch gekennzeichnet, dass man Malonat und/oder Pyruvat in das Bestimmungsmedium einführt.

24. Verfahren nach Anspruch 17 zur Bstimmung von Brenztraubensäure oder ihrer Salze mit einer Vorrichtung nach Anspruch 9, dadurch gekennzeichnet, dass man L-Lactat in das Bestimmungsmedium einführt.

25. Verfahren nach Anspruch 17 zur Bestimmung von Fumarsäure oder ihrer Salze in Gegenwart anderer Substrate der Atmungskette, die stören können, mit einer Vorrichtung nach Anspruch 12, dadurch gekennzeichnet, dass man Pyruvat in das Bestimmungsmedium einführt.

## Claims

1. Device for the specific assay of substrates, inhibitors, or activators of the cellular oxidation chain, characterized in that it contains an oxygen sensor, associated with means for measuring the oxygen detected, and the transporting species of the cellular respiratory chain in the form of immobilized bacteria, of immobilized microorganisms or of their immobilized membrane preparations, these cells having been cultured beforehand on a medium whose composition has produced a modification of the cellular synthesis of the flavoprotein corresponding to the compound to be assayed.

2. Device according to claim 1, characterized in that the effectors to be assayed are chosen from D-lactic and L-lactic, pyruvic, succinic, fumaric, oxaloacetic and L-malic acids and their salts, NADH and NADPH, or alternatively glutamic, aspartic, citric, isocitric, ascorbic, 3-glycerophosphoric and oxalic acids and their salts, or alternatively oxidoreductases dependent on $NAD^+$ or $NADP^+$ cofactors.

3. Device according to one of claims 1 and 2, characterized in that the medium for culturing the cells has induced in that the medium for culturing the cells has induced the synthesis of the flavoprotein.

4. Device according to claim 3, characterized in that the induction results from the presence in the minimum culture medium of only one source of carbon, which is the substrate of the respiratory chain corresponding to the flavoprotein.

5. Device according to claim 1, characterized in that the culture medium contains glucose in order to repress certain flavoproteins.

6. Device according to any one of the preceding claims, characterized in that the immobilization takes place in gelatin with a tanning by the action of glutaraldehyde.

7. Device according to claim 6, characterized in that the cells are immobilized in a film prepared from a cell suspension containing 10 to 40 mg of bacterial proteins and 50 mg of gelatin per ml, spread over 40 cm$^2$, dried and then tanned by the action of an aqueous solution of glutaraldehyde having a concentration of between 1 and 4% (w/v) for a few minutes.

8. Device according to one of the preceding claims, characterized in that it consists of an oxygen electrode on which the film of cells or immobilized cell preparations has been deposited.

9. Device according to one of claims 1 to 4 or 6 to 8 for assay of L-lactic acid, pyruvic acid or their salts, characterized in that it contains immobilized bacteria derived from a minimum culture medium containing a lactate as the only source of carbon.

10. Device according to one of claims 1 to 4 or 6 to 8 for the assay of succinic acid, fumaric acid or their salts, characterized in that the bacteria have been cultured on a minimum medium containing a succinate as the only source of carbon.

11. Device according to claim 10 for the assay of succinic acid or its salts in the presence of other substrates of the respiratory chain, characterized in that Escherichia coli is subjected to a temperature of 53 °C to 57 °C, for 1 to 2 hours, before immobilization.

12. Device according to one of claims 1 to 4 or 6 to 8 for the assy of L-malic acid or oxaloacetic or fumaric acid or their salts, characterized in that the bacteria have been cultured on a minimum medium containing a malate as the only source of carbon.

13. Device according to one of claims 5 to 8 for the assay fo D-lactic acid or its salts, characterized in that the bacteria have been cultured on a medium containing glucose.

14. Device according to one of claims 5 to 8 for the assay of pyruvic acid or its salts, NADH or NADPH, characterized in that the bacteria originate from a culture medium containing glucose and in that the membranes of the bacteria have been rendered permeable before their immobilization.

15. Device according to claim 14 for the assay of pyruvic acid or its salts in the presence of other substrates of the respiratory chain, characterized in that Escherichia coli are subjected to a temperature of 53 °C to 57 °C, for 1 to 2 hours, before their membranes are rendered permeable.

16. Device according to one of claims 1 to 4 or 6 to 8 for the assay of 3-glycerophosphoric or pyruvic acid, characterized in that the bacteria have been cultured on a minimum medium containing glycerol as the only source of carbon.

17. Method for the specific assay of substrates, inhibitores and activators of the cellular oxidation chain, characterized in that the amount of oxygen consumed by the system of transporting species forming the respiratory chain, in order to convert the product to be assayed, is determined, the system being introduced into the test medium in the form of immobilized bacteria, of immobilized microorganisms or of one of their immobilized membrane preparations, these cells having been cultured beforehand in media whose composition has brought about a modification of the cellular synthesis of the flavoprotein corresponding to the compound to be assayed.

18. Method according to claim 17, characterized in that the effetors to be assayed are chosen from D-lactic and L-lactic, pyruvic, succinic, fumaric, oxaloacetic and L-malic acids and their salts, NADH and NADPH, or alternatively glutamic, aspartic, citric, isocitric, ascorbic, 3-glycerophosphoric and oxalic acids and their salts, or alternatively oxidoreductases dependent on $NAD^+$ and $NADP^+$ cofactors.

19. Assay method according to claim 17, employing a device according to any one of claims 1 to 16.

20. Method according to claim 17 for the assay of L-lactic acid or its salts in the presence of other substrates, capable of interfering, of the respiratory chain, with a device according to claim 9, characterized in that fumarate or malonate is introduced into the assay medium.

21. Method according to claim 17 for the assay of D-lactic acid or its salts in the presence of other substrates, capable of interfering, of the respiratory chain, with a device according to claim 13, characterized in that fumarate or malonate is introduced into the assay medium.

22. Method according to claim 17 for the assay of succinic acid or its salts in the presence of other substrates, capable of interfering, of the respiratory chain, with a device according to claim 11, characterized in that pyruvate is introduced into the assay medium.

23. Method according to claim 17 for the assay of L-malic acid or its salts in the presence of other substrates, capable of interfering, of the respiratory chain, with a device according to claim 12, characterized in that malonate and/or pyruvate is/are introduced into the assay medium.

24. Method according to claim 17 for the assay of pyruvic acid or its salts with a device according to claim 9, characterized in that L-lactate is introduced into the assay medium.

25. Method according to claim 17 for the assay of fumaric acid or its salts in the presence of other substrates, capable of interfering, of the respiratory chain, with a device according to claim 12, characterized in that pyruvate is introduced into the assay medium.

FIG.1

FIG.2

FIG.3

FIG.4